# FASCICULE DE BREVET EUROPEEN

(11) **EP 1 501 569 B1**
(45) Date de publication et mention de la délivrance du brevet: **02.12.2009**
(21) Numéro de dépôt: 03749927.4
(22) Date de dépôt: 06.05.2003
(51) Int. Cl.: A61M 5/00, B65D 81/20, A61L 2/26, A61L 2/20, B65B 55/10, B65B 31/02

(54) **EMBALLAGE DESTINE A ETRE UTILISE POUR TRANSPORTER DES OBJETS STERILES OU A STERILISER**
VERPACKUNG FÜR DEN TRANSPORT VON STERILEN GEGENSTÄNDEN ODER ZU STERILISIERENDEN GEGENSTÄNDEN
PACKAGING FOR THE TRANSPORT OF STERILE OBJECTS OR OBJECTS TO BE STERILISED

(30) Priorité: 07.05.2002 FR 0205728
(43) Date de publication de la demande: 02.02.2005
(73) Titulaire: Becton Dickinson France, 38800 Le Pont de Claix (FR)
(72) Inventeur: Raynal-Olive, Claire, 38450 Vif (FR); Grimard, Jean-Pierre, 38450 Vif (FR)
(74) Mandataire: Brédeville, Odile Marie
(86) Numéro de dépôt international: PCT/FR2003/001407
(87) Numéro de publication internationale: WO 2003/094999

(56) Documents cités:
- EP-A- 0 266 688
- EP-A- 0 307 173
- EP-A- 0 846 445
- DE-B- 1 586 778
- US-A- 3 302 859
- US-A- 3 503 497
- US-A- 5 868 244

## Description

La présente invention concerne un emballage destiné à être utilisé pour transporter des objets stériles ou à stériliser, un procédé de fabrication de cet emballage, et l'utilisation de cet emballage dans un procédé de décontamination. L'emballage selon l'invention peut notamment être utilisé pour transporter des composants de seringues, en particulier des corps de seringue destinés à être ultérieurement remplis par un produit actif ou un médicament.

Les conditions de stérilité dans lesquelles doivent se dérouler certaines étapes de manipulation ou de transport d'objets destinés à un usage médical sont très contraignantes, en particulier dans l'industrie pharmaceutique. Il est donc d'une grande importance de réaliser des emballages compatibles avec de telles exigences.

Dans la suite de la description, il sera fait mention d'un matériau sélectivement étanche qu'il convient de définir. Par l'expression "sélectivement étanche" telle qu'utilisée dans la présente description ainsi que dans les revendications, on entend que le matériau est conçu, en termes de structure, de manière à contrôler tout échange de l'intérieur de l'emballage avec son environnement extérieur. Ceci signifie entre autres que l'emballage est étanche, individuellement ou en combinaison, à la contamination par des microorganismes, bactéries et/ou un matériau biologiquement actif, susceptibles de venir en contact avec l'emballage lors de sa manipulation, tout en restant perméable aux gaz en général. Le degré de perméabilité aux gaz peut varier en fonction de la nature du gaz. De préférence, le matériau sélectivement étanche permettra le passage aisé d'un gaz de stérilisation, comme par exemple l'oxyde d'éthylène (ETO), et limitera le passage d'un gaz de décontamination, comme par exemple des vapeurs de peroxyde d'hydrogène.

Il est connu de placer des objets stériles ou à stériliser dans une boîte en matière plastique (voir EP0266688) de fixer ensuite une feuille de couverture en matériau sélectivement étanche sur cette boîte de manière à sceller cette dernière, de placer la boîte ainsi scellée dans un deuxième emballage comprenant une fenêtre fermée par une feuille en matériau sélectivement étanche, et de procéder à une stérilisation de l'ensemble par un gaz du type ETO. L'emballage ainsi stérilisé est placé dans une boîte en carton pour son expédition ; à destination, il est procédé à l'ouverture de la boîte en carton et dudit deuxième emballage, puis à la décontamination de ladite boîte en matière plastique et à l'ouverture de cette boîte.

Dans le cas de composants de seringues, il est connu d'utiliser une boîte notamment en polystyrène et une feuille de couverture en matériau commercialisé sous la marque TYVEK®, scellé sur la boîte. Ce matériau est formé à base de filaments de PEHD (polyéthylène haute densité) ou autre polymère, liés notamment par l'intermédiaire de chaleur et de pression.

Pour ledit deuxième emballage, il est connu d'utiliser un sac en matière plastique, la feuille de fermeture de la fenêtre que comprend ce sac étant également en "TYVEK®".

A destination, après retrait de ce deuxième emballage, la boîte est exposée à un gaz de décontamination, par exemple à des vapeurs de peroxyde d'hydrogène, afin de réaliser sa décontamination. Cette exposition se fait dans un sas ou un tunnel d'acheminement de cette boîte à une zone stérile.

Ce type de décontamination est bien adapté à certaines utilisations, notamment à la décontamination d'emballages contenant des corps de seringues tels que précités. La demanderesse a toutefois pu constater que dans certains cas, il existait des interactions indésirables entre les objets contenus dans l'emballage, en particulier des corps de seringue, et les produits avec lesquels ces objets sont ensuite en contact, en particulier des produits actifs ou des médicaments venant ultérieurement remplir les corps de seringue. Ce phénomène est apparu exister d'autant plus que du "TYVEK®" était utilisé en tant que matériau sélectivement étanche.

L'invention vise à remédier à cet inconvénient important. Son objectif est donc de fournir un emballage pour des objets stériles ou à stériliser, pouvant être décontaminé au moyen d'un gaz de décontamination, par exemple au moyen de vapeurs de peroxyde d'hydrogène, sans qu'il existe ultérieurement des interactions indésirables entre les objets contenus dans l'emballage, en particulier des corps de seringue, et des produits avec lesquels ces objets sont ultérieurement destinés à être en contact, en particulier des produits actifs ou des médicaments venant ultérieurement remplir des corps de seringue.

L'objectif de la présente invention est également de fournir un procédé pour la réalisation de cet emballage.

L'emballage concerné comprend, de manière connue en soi, une boîte destinée à recevoir les objets stériles ou à stériliser et une feuille de couverture, fixée sur la boîte de manière à sceller cette dernière de façon étanche.

Selon l'invention, ladite feuille de couverture est en un matériau non perméable à un gaz de décontamination, par exemple à des vapeurs de peroxyde d'hydrogène, et comprend au moins une fenêtre fermée par une pièce de matériau sélectivement étanche et l'emballage comprend en outre au moins une pièce flexible d'un matériau non perméable à un gaz de décontamination, par exemple à des vapeurs de peroxyde d'hydrogène, ladite pièce flexible étant fixée à la feuille de couverture par au moins un de ses bords et comprenant une partie libre, la partie libre de ladite pièce étant mobile entre une position de diffusion, permettant une diffusion non restreinte d'un gaz de stérilisation à l'intérieur de la boîte au travers de la fenêtre, et une position de non diffusion, minimisant, voire empêchant, la diffusion du gaz de décontamination, par exemple des vapeurs de peroxyde d'hydrogène, à l'intérieur de la boîte au travers de la fenêtre.

Le procédé de fabrication de l'emballage selon l'invention comprend les étapes consistant à :
- former une feuille de couverture en un matériau non perméable à un gaz de décontamination, par exemple aux vapeurs de peroxyde d'hydrogène, en aménageant au moins une fenêtre dans cette feuille de couverture ;
- placer une pièce d'un matériau sélectivement étanche dans ladite fenêtre de manière à clore cette fenêtre au moyen de ce matériau,
- fixer une pièce flexible d'un matériau non perméable à un gaz de décontamination, par exemple à des vapeurs de peroxyde d'hydrogène, sur la feuille de couverture, ladite pièce flexible étant fixée à la feuille de couverture par au moins un de ses bords et comprenant une partie libre, ladite partie libre étant positionnée en regard de la fenêtre,
- placer les objets à emballer dans une boîte, et
- fixer ladite feuille de couverture sur la boîte de manière à sceller cette boîte de façon étanche.

L'invention concerne également l'utilisation de l'emballage précité dans un procédé de décontamination de cet emballage par un gaz de décontamination, par exemple par des vapeurs de peroxyde d'hydrogène.

L'invention concerne encore un procédé de stérilisation et de décontamination utilisant l'emballage ci-dessus, caractérisé en ce qu'il comprend les étapes consistant à :
- placer l'emballage en position de diffusion au cours du processus de stérilisation ; et
- placer l'emballage en position de non diffusion au cours du processus de décontamination.

La demanderesse a en effet pu constater que des résidus de peroxyde d'hydrogène se retrouvaient sur les objets contenus dans la boîte lorsque la pièce en matériau sélectivement étanche n'est pas étanche aux vapeurs de peroxyde d'hydrogène, comme cela s'avère être le cas du "TYVEK®", et que ces résidus étaient à l'origine des interactions indésirables précitées. Ces interactions se produisent d'autant plus dans le cas de corps de seringue, lesdits résidus s'accumulant dans ceux-ci du fait que les vapeurs de peroxyde d'hydrogène sont plus lourdes que l'air contenu dans l'emballage.

L'invention solutionne ce problème en prévoyant une feuille de couverture en un matériau non perméable à un gaz de décontamination, par exemple aux vapeurs de peroxyde d'hydrogène, qui permet de protéger efficacement les objets contenus dans la boîte à l'égard de ce gaz de décontamination, par exemple des vapeurs de peroxyde d'hydrogène, mais présentant au moins une fenêtre fermée par une pièce de matériau sélectivement étanche pour permettre au gaz de stérilisation de pénétrer à l'intérieur de cette boîte, une pièce flexible d'un matériau non perméable à un gaz de décontamination, par exemple à des vapeurs de peroxyde d'hydrogène, étant fixée à la feuille de couverture par au moins un de ses bords, en regard de la fenêtre. Grâce à sa partie libre et à sa flexibilité, la pièce flexible joue le rôle d'une valve. Ainsi, dans une position de diffusion, la partie libre de cette pièce flexible, du fait de la gravité, est détachée de la feuille de couverture. De ce fait, elle ne recouvre pas la fenêtre en regard de laquelle elle est fixée et elle laisse libre cette fenêtre pour le passage du gaz de stérilisation au travers de cette fenêtre vers l'intérieur de la boîte et les objets à stériliser. Au contraire, dans une position de non diffusion, par exemple lorsque l'emballage a été placé dans une position renversée par rapport à la position de diffusion, la partie libre de la pièce flexible recouvre la fenêtre et minimise, voire empêche, le passage du gaz de décontamination au travers de cette fenêtre.

La feuille de couverture est en un matériau non perméable à un gaz de décontamination, par exemple à des vapeurs de peroxyde d'hydrogène. Par « matériau non perméable à un gaz de décontamination », on entend, au sens de la présente invention, un matériau minimisant, voire empêchant, la diffusion d'un gaz de décontamination, par exemple des vapeurs de peroxyde d'hydrogène, au travers de ce matériau. De préférence, ce matériau est choisi parmi les polymères flexibles ou non, les polyoléfines, telles que le polyéthylène ou le polypropylène, le polyester, le polyamide et leurs combinaisons.

De préférence, la pièce en matériau sélectivement étanche est en matériau poreux. De préférence, ce matériau sélectivement étanche est choisi parmi le papier, les matériaux à base de fibres naturelles, par exemple végétales, ou synthétiques, les matériaux à base de filaments de polyéthylène à haute densité, ou d'autre polymère, liés notamment par l'intermédiaire de chaleur et de pression, et leurs combinaisons. De préférence encore, le matériau sélectivement étanche est un matériau à base de filaments de polyéthylène à haute densité liés par l'intermédiaire de chaleur et de pression, en particulier le matériau commercialisé sous la marque TYVEK® par la société Du Pont de Nemours.

La pièce flexible peut être fixée sur la feuille de couverture par exemple par collage ou par soudure.

Dans une forme de réalisation de l'invention, la boîte comprend un rebord dépassant de la feuille de couverture et la pièce flexible est fixée sur la face externe de la feuille de couverture.

Dans une autre forme de réalisation de l'invention, la pièce flexible est fixée sur la face interne de la feuille de couverture.

Avantageusement, la pièce flexible est fixée sur la feuille de couverture en regard de la fenêtre. De préférence, la pièce flexible présente une forme similaire à celle de la fenêtre, la surface de la pièce flexible étant supérieure à celle de la fenêtre de telle façon qu'en position de non diffusion, la pièce flexible recouvre avec débordement cette fenêtre sur tout son périmètre.

Dans une forme préférée de réalisation de l'invention, la feuille de couverture comprend plusieurs fenêtres fermées chacune par une pièce de matériau sélectivement étanche. Dans ce cas, la pièce flexible peut être fixée sur la feuille de couverture en regard de plusieurs fenêtres, de telle façon qu'en position de non diffusion, la pièce flexible recouvre avec débordement l'ensemble de ces fenêtres.

Alternativement, plusieurs pièces flexibles peuvent être fixées sur la feuille de couverture, une en regard de chaque fenêtre, de telle façon qu'en position de non diffusion, chaque pièce flexible recouvre avec débordement la fenêtre en regard de laquelle elle est fixée.

La pièce flexible est en un matériau non perméable à un gaz de décontamination, par exemple des vapeurs de peroxyde d'hydrogène. De préférence, ce matériau est choisi parmi les polymères flexibles ou non, les polyoléfines, telles que le polyéthylène ou le polypropylène, le polyester, le polyamide et leurs combinaisons.

La boîte de l'emballage selon l'invention est réalisée de préférence en un matériau plastique rigide ou semi-rigide. De préférence, ce matériau est choisi parmi le polystyrène, le polypropylène, le polycarbonate, le polyester, le chlorure de polyvinyle et leurs combinaisons.

L'emballage peut comprendre en outre, à l'intérieur de la boîte, au moins une couche limitant le passage d'un gaz de décontamination, par exemple de vapeurs de peroxyde d'hydrogène ou à même d'absorber un gaz de décontamination, par exemple des vapeurs de peroxyde d'hydrogène.

Ces couches permettent ainsi de limiter l'introduction de ces vapeurs de peroxyde d'hydrogène dans la boîte ou d'absorber les vapeurs de peroxyde d'hydrogène qui auraient pu s'introduire dans cette boîte.

Cette couche peut avoir une forme et des dimensions telles qu'elle puisse être placée le long de la feuille de couverture et qu'elle s'étende, dans cette position, entre la feuille de couverture et les objets contenus dans l'emballage.

Dans le cas où la pièce flexible est fixée sur la face externe de la feuille de couverture, ladite couche ou au moins une desdites couches peut être rapportée sur la feuille de couverture notamment par collage ou soudure ; cette ou ces couches sont alors dimensionnées de manière à délimiter sur la feuille de couverture une zone périphérique de fixation de cette feuille de couverture à la boîte.

Ladite couche ou au moins une desdites couches peuvent également être simplement disposées sur les objets placés à l'intérieur de la boîte, préalablement au scellage de la feuille de couverture, ou sur des appuis prévus à cet effet, ou sur une pièce de positionnement des objets, placée dans cette boîte.

Dans le cas où la pièce flexible est fixée sur la face externe de la feuille de couverture, l'emballage peut également comprendre au moins une desdites couches rapportée sur la feuille de couverture et au moins une autre desdites couches disposée à l'intérieur de la boîte.

Ladite couche peut être un matériau à base de filaments de polyéthylène à haute densité, ou autre polymère, liés notamment par l'intermédiaire de chaleur et de pression, et en particulier le matériau commercialisé sous la marque TYVEK®.

Ladite couche peut également être en un matériau à base de fibres naturelles, par exemple végétales, ou comprendre une feuille métallisée ou métallique, un matériau plastique, ou comprendre au moins deux feuilles complémentaires de matériau sélectivement étanche.

L'invention sera mieux comprise à l'aide du dessin annexé dans lequel :
- la figure 1 est une vue partielle en coupe longitudinale selon une première forme de réalisation, dans une position dans laquelle l'emballage est placé au cours du processus de stérilisation au moyen d'un gaz de stérilisation, par exemple par de l'oxyde d'éthylène (ETO) ;
- la figure 2 est une vue similaire à la figure 1, dans une position renversée par rapport à la figure 1, dans laquelle l'emballage est placé au cours d'un processus ultérieur de décontamination au moyen d'un gaz de décontamination, par exemple au moyen de vapeurs de peroxyde d'hydrogène (VHP) ;
- la figure 3 est une vue partielle en coupe longitudinale selon une deuxième forme de réalisation dans une position dans laquelle l'emballage est placé au cours dudit processus de stérilisation, la feuille de couverture comprenant deux fenêtres,
- la figure 4 est une vue similaire à la figure 3, dans une position renversée par rapport à la figure 3, dans laquelle l'emballage est placé au cours dudit processus de décontamination.

Par simplification, les parties ou éléments de la première forme de réalisation qui se retrouvent de manière identique ou similaire dans la deuxième forme de réalisation seront désignés par les mêmes références numériques et ne seront pas redécrits en détails.

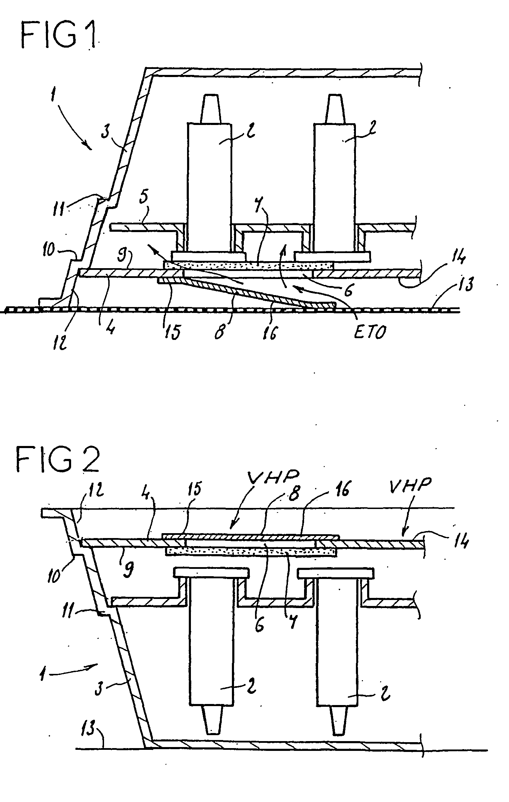

Les figures 1 et 2 représentent un emballage 1 utilisé pour transporter des composants de seringues, en particulier, dans l'exemple représenté, des corps de seringue 2 destinés à être ultérieurement remplis par un produit actif ou un médicament.

L'emballage 1 comprend une boîte 3, un plateau 5 supportant les corps de seringues 2 et une feuille de couverture 4 fixée sur la boîte 3 de manière à sceller cette dernière de façon étanche.

La boîte 3 est en polystyrène et comprend une bride périphérique 10 permettant le scellage de la feuille 4. Elle forme également un épaulement 11 de réception du plateau 5. La boîte 3 comporte également un rebord 12 dépassant de la feuille de couverture 4. Sur la figure 1, la boîte 3 repose sur un support non continu, ici une grille 13, dans une position dans laquelle les corps de seringue 2 sont en position collerette en bas.

La feuille de couverture 4 est en un matériau non perméable à un gaz de décontamination, et elle comporte une fenêtre 6 fermée par une pièce 7 en « Tyvek® », qui est un matériau sélectivement étanche. Cette pièce 7 est soudée sur la face interne 9 de la feuille de couverture 4. En regard de la fenêtre 6, une pièce flexible 8 est fixée sur la face externe 14 de la feuille de couverture 4. Cette pièce flexible 8 est soudée sur la feuille de couverture 4 par un de ses bords 15 et comporte une partie libre 16. Du fait de la présence du rebord 12, de la flexibilité de la pièce 8 et de la gravité, la partie libre 16 repose sur la grille 13 sur laquelle est placé l'emballage 1.

Ainsi, dans cette position de diffusion, représentée à la figure 1, on procède à l'étape de stérilisation. Le gaz de stérilisation, matérialisé par ETO et les flèches correspondantes sur la figure 1, diffuse à travers la grille 13 puis à travers la fenêtre 6 et la pièce 7 en matériau sélectivement étanche vers l'intérieur de la boîte 3, baignant ainsi les corps de seringues 2 à stériliser.

Sur la figure 2 est représentée la position de non diffusion. L'emballage est en position renversée par rapport à la position de la figure 1. Du fait de la flexibilité de la pièce flexible 8 et de la gravité, la partie libre 16 de cette pièce flexible 8 recouvre entièrement la fenêtre 6 fermée par la pièce 7 en matériau sélectivement étanche. Dans cette position, la pièce flexible 8 minimise, voire empêche, la diffusion du gaz de décontamination, c'est-à-dire des vapeurs de peroxyde d'hydrogène matérialisées par VHP et les flèches correspondantes sur la figure 2.

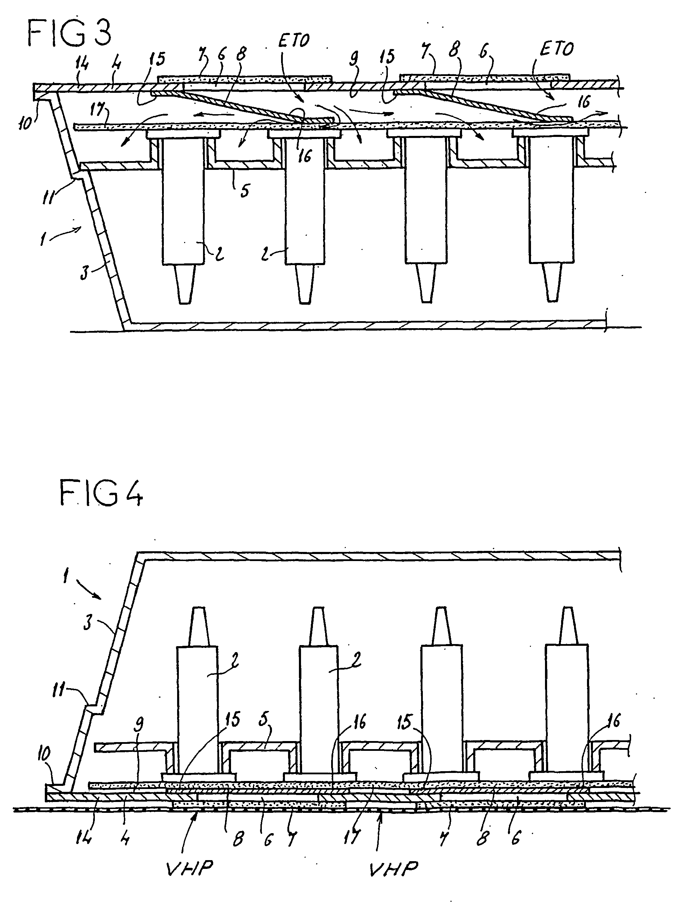

Dans la deuxième forme de réalisation montrée sur les figures 3 et 4, la boîte 3 ne comprend pas de rebord dépassant de la feuille de couverture 4 et la feuille de couverture 4 comprend deux fenêtres 6 fermées chacune par une pièce 7 en matériau sélectivement étanche soudée sur la face externe 14 de la feuille de couverture 4. En regard de chaque fenêtre 6, est soudée une pièce flexible 8 par un de ses bords 15, sur la face interne 9 de la feuille de couverture 4.

L'emballage 1 comprend également une couche 17 en Tyvek®, qui est un matériau sélectivement étanche. Cette couche 17 est positionnée entre les corps de seringues 2 et la feuille de couverture 4.

Dans la position de diffusion, représentée à la figure 3, du fait de la flexibilité de la pièce flexible 8 et de la gravité, la partie libre 16 de la pièce flexible 8 repose sur la couche 17 en matériau sélectivement étanche et laisse libre passage au gaz de stérilisation au travers de la fenêtre 6.

Dans cette position, on procède à la stérilisation de l'emballage à l'aide du gaz de stérilisation, ici l'oxyde d'éthylène matérialisé par ETO et les flèches correspondantes sur la figure 3. L'oxyde d'éthylène diffuse à l'intérieur de la boîte 3 au travers des fenêtres 6 et de la couche 17 en matériau sélectivement étanche et baigne les corps de seringues 2 à stériliser.

Sur la figure 4 est représentée la position de non diffusion. L'emballage 1 est en position renversée par rapport à la position de la figure 3. Pour chaque fenêtre 6, du fait de la flexibilité de la pièce flexible 8 et de la gravité de l'ensemble constitué par le plateau 5, les corps de seringues 2 et la couche 17, la partie libre 16 de la pièce flexible 8 recouvre entièrement la fenêtre 6 fermée par la pièce 7 en matériau sélectivement étanche. Dans cette position, les pièces flexibles 8 minimisent, voire empêchent, la diffusion du gaz de décontamination, c'est-à-dire des vapeurs de peroxyde d'hydrogène matérialisées par VHP et les flèches correspondantes sur la figure 4.

L'invention apporte une amélioration déterminante à la technique antérieure,en fournissant un emballage qui est efficace à l'égard d'éventuelles pénétrations d'un gaz de décontamination, par exemple de vapeurs de peroxyde d'hydrogène, lors du processus de décontamination sans amoindrissement significatif de l'aptitude de cet emballage à être stérilisé au moyen d'un gaz de stérilisation. L'invention fournit également un procédé amélioré de stérilisation et de décontamination de cet emballage.

L'invention n'est pas limitée aux formes de réalisation décrites ci-dessus à titre d'exemples.

## Revendications

1. Emballage (1) destiné à être utilisé pour transporter des objets stériles ou à stériliser, comprenant une boîte (3) destinée à recevoir les objets stériles ou à stériliser et une feuille de couverture (4) fixée sur la boîte de manière à sceller cette dernière de façon étanche ;
emballage **caractérisé en ce que** ladite feuille de couverture est en un matériau non perméable à un gaz de décontamination, par exemple à des vapeurs de peroxyde d'hydrogène, et comprend au moins une fenêtre (6) fermée par une pièce (7) de matériau sélectivement étanche, et **en ce qu'**il comprend en outre au moins une pièce flexible (8) d'un matériau non perméable à un gaz de décontamination, par exemple à des vapeurs de peroxyde d'hydrogène, ladite pièce flexible (8) étant fixée à la feuille de couverture (4) par au moins un de ses bords (15) et comprenant une partie libre (16), la partie libre (16) de ladite pièce étant mobile entre une position de diffusion, permettant une diffusion non restreinte d'un gaz de stérilisation à l'intérieur de la boîte (3) au travers de la fenêtre (6), et une position de non diffusion, minimisant, voire empêchant, la diffusion du gaz de décontamination, par exemple des vapeurs de peroxyde d'hydrogène, à l'intérieur de la boîte (3) au travers de la fenêtre (6).

2. Emballage selon la revendication 1, **caractérisé en ce que** le matériau sélectivement étanche est choisi parmi le papier, les matériaux à base de fibres naturelles ou synthétiques, les matériaux à base de filaments de polyéthylène à haute densité, ou d'autre polymère, liés notamment par l'intermédiaire de chaleur et de pression, et leurs combinaisons.

3. Emballage selon la revendication 2, **caractérisé en ce que** le matériau sélectivement étanche est un matériau à base de filaments de polyéthylène à haute densité liés par l'intermédiaire de chaleur et de pression.

4. Emballage selon l'une quelconque des revendications précédentes, **caractérisé en ce que** la boîte (3) comprend un rebord (12) dépassant de la feuille de couverture (4) et la pièce flexible (8) est fixée sur la face externe (14) de la feuille de couverture.

5. Emballage selon l'une des revendications 1 à 3, **caractérisé en ce que** la pièce flexible (8) est fixée sur la face interne (9) de la feuille de couverture.

6. Emballage selon l'une quelconque des revendications précédentes, **caractérisé en ce que** la pièce flexible (8) est fixée sur la feuille de couverture (4) en regard de la fenêtre (6).

7. Emballage selon l'une quelconque des revendications précédentes, **caractérisé en ce que** la pièce flexible (8) présente une forme similaire à celle de la fenêtre (6), la surface de la pièce flexible (8) étant supérieure à celle de la fenêtre (6), de telle façon qu'en position de non diffusion, la pièce flexible (8) recouvre avec débordement cette fenêtre (6) sur tout son périmètre.

8. Emballage selon l'une quelconque des revendications précédentes, **caractérisé en ce que** la feuille de couverture (4) comprend plusieurs fenêtres (6) fermées chacune par une pièce (7) de matériau sélectivement étanche.

9. Emballage selon la revendication 8, **caractérisé en ce que** la pièce flexible (8) est fixée sur la feuille de couverture (4) en regard de plusieurs fenêtres (6) de telle façon qu'en position de non diffusion, la pièce flexible (8) recouvre avec débordement l'ensemble de ces fenêtres (6).

10. Emballage selon la revendication 8, **caractérisé en ce que** plusieurs pièces flexibles (8) sont fixées sur la feuille de couverture (4), une en regard de chaque fenêtre (6) de telle façon qu'en position de non diffusion, chaque pièce flexible (8) recouvre avec débordement la fenêtre (6) en regard de laquelle elle est fixée.

11. Emballage selon l'une quelconque des revendications précédentes, **caractérisé en ce qu'**il comprend, à l'intérieur de la boîte, au moins une couche (17) limitant le passage d'un gaz de décontamination, par exemple de vapeurs de peroxyde d'hydrogène, ou à même d'absorber un gaz de décontamination, par exemple des vapeurs de peroxyde d'hydrogène.

12. Emballage selon la revendication 11, **caractérisé en ce que** ladite couche a une forme et des dimensions telles qu'elle puisse être placée le long de la feuille de couverture et qu'elle s'étende, dans cette position, entre la feuille de couverture (4) et les objets (2) contenus dans l'emballage.

13. Emballage selon l'une des revendications 11 à 12, **caractérisé en ce que** ladite couche ou au moins une desdites couches sont disposées sur les objets placés à l'intérieur de la boîte, préalablement au scellage de la feuille de couverture, ou sur des appuis prévus à cet effet, ou sur une pièce de positionnement des objets, placée dans cette boîte.

14. Procédé de fabrication de l'emballage (1) selon l'une des revendications 1 à 13, **caractérisé en ce qu'**il comprend les étapes consistant à :
- former une feuille de couverture (4) en un matériau non perméable à un gaz de décontamination, par exemple aux vapeurs de peroxyde d'hydrogène, en aménageant au moins une fenêtre (6) dans cette feuille de couverture ;
- placer une pièce (7) d'un matériau sélectivement étanche dans ladite fenêtre de manière à clore cette fenêtre au moyen de ce matériau,
- fixer une pièce flexible (8) d'un matériau non perméable à un gaz de décontamination, par exemple à des vapeurs de peroxyde d'hydrogène, sur la feuille de couverture, ladite pièce flexible (8) étant fixée à la feuille de couverture (4) par au moins un de ses bords (15) et comprenant une partie libre (16), ladite partie libre (16) étant positionnée en regard de la fenêtre (6),
- placer les objets (2) à emballer dans une boîte (3), et
- fixer ladite feuille de couverture (4) sur la boîte (3) de manière à sceller cette boîte de façon étanche..

15. Procédé selon la revendication 14, **caractérisé en ce que** la pièce flexible (8) est fixée sur la face externe de la feuille de couverture (4).

16. Procédé selon la revendication 14, **caractérisé en ce que** la pièce flexible (8) est fixée sur la face interne de la feuille de couverture (4).

17. Utilisation de l'emballage (1) selon l'une des revendications 1 à 13 dans un procédé de décontamination de cet emballage par un gaz de décontamination, par exemple par des vapeurs de peroxyde d'hydrogène.

18. Utilisation de l'emballage (1) selon l'une des revendications 1 à 13 pour transporter des composants de seringues, en particulier des corps de seringue (2) destinés à être ultérieurement remplis par un produit actif ou un médicament.

19. Procédé de stérilisation et de décontamination utilisant un emballage (1) selon l'une quelconque des revendications 1 à 13, **caractérisé en ce qu'**il comprend les étapes consistant à :
- placer l'emballage (1) en position de diffusion au cours du processus de stérilisation ; et
- placer l'emballage (1) en position de non diffusion au cours du processus de décontamination.

## Claims

1. A packaging (1) intended to be used for transporting sterile objects or objects that are to be sterilized, comprising a box (3) intended to house the sterile objects or objects that are to be sterilized, and a cover sheet (4) fixed to the box in such a way as to seal the latter imperviously;
which packaging is **characterized in that** said cover sheet is made of a material that is not permeable to a decontamination gas, for example to hydrogen peroxide vapors, and comprises at least one window (6) closed by a piece (7) of selectively impervious material, and **in that** it further comprises at least one flexible piece (8) of a material that is not permeable to a decontamination gas, for example to hydrogen peroxide vapors, the said flexible piece (8) being fixed to the cover sheet (4) by at least one of its edges (15) and comprising a free part (16), the free part (16) of said piece being able to move between a diffusion position, allowing unrestricted diffusion of sterilization gas into the box (3) through the window (6) and a non-diffusion position minimizing or even preventing the diffusion of decontamination gas, for example of hydrogen peroxide vapors, into the box (3) through the window (6).

2. The packaging as claimed in claim 1, **characterized in that** the selectively impervious material is chosen from paper, materials based on natural or synthetic fibers, materials based on filaments of high density polyethylene, or some other polymer, these being bound in particular by heat and pressure, and combinations thereof.

3. The packaging as claimed in claim 2, **characterized in that** the selectively impervious material is a material based on filaments of high density polyethylene bound by heat and pressure.

4. The packaging as claimed in any one of the preceding claims, **characterized in that** the box (3) has a rim (12) extending beyond the cover sheet (4) and the flexible piece (8) is fixed to the exterior face (14) of the cover sheet.

5. The packaging as claimed in one of claims 1 to 3, **characterized in that** the flexible piece (8) is fixed to the interior face (9) of the cover sheet.

6. The packaging as claimed in any one of the preceding claims, **characterized in that** the flexible piece (8) is fixed to the cover sheet (4) facing the window (6).

7. The packaging as claimed in any one of the preceding claims, **characterized in that** the flexible piece (8) has a shape similar to that of the window (6), the area of the flexible piece (8) being greater than that of the window (6) so that in the non-diffusion position, the flexible piece (8) covers this window (6) with overlap around its entire periphery.

8. The packaging as claimed in any one of the preceding claims, **characterized in that** the cover sheet (4) has several windows (6) each closed by a piece (7) of selectively impervious material.

9. The packaging as claimed in claim 8, **characterized in that** the flexible piece (8) is fixed to the cover sheet (4) facing several windows (6) so that in the non-diffusion position, the flexible piece (8) covers all the windows (6) with overlap.

10. The packaging as claimed in claim 8, **characterized in that** several flexible pieces (8) are fixed to the cover sheet (4), one facing each window (6) so that in the non-diffusion position, each flexible piece (8) covers, with an overlap, the window (6) facing which it is fixed.

11. The packaging as claimed in any one of the preceding claims, **characterized in that** it comprises, inside the box, at least one layer (17) limiting the passage of a decontamination gas, for example of hydrogen peroxide vapors, or able to absorb a decontamination gas, for example hydrogen peroxide vapors.

12. The packaging as claimed in claim 11, **characterized in that** the said layer has shape and size such that it can be placed along the cover sheet and that, in this position, it extends between the cover sheet (4) and the objects (2) contained in the packaging.

13. The packaging as claimed in one of claims 11 to 12, **characterized in that** said layer or at least one of said layers are arranged over the objects placed inside the box prior to the cover sheet being sealed, or on supports provided for that purpose, or on an objects-positioning piece placed in this box.

14. A method of manufacturing the packaging (1) as claimed in one of claims 1 to 13, **characterized in that** it comprises the steps consisting in:
- forming a cover sheet (4) made of a material that is not permeable to a decontamination gas, for example to hydrogen peroxide vapors, while forming at least one window (6) in this cover sheet;
- placing a piece (7) of a selectively impervious material in said window in such a way as to close this window using this material,
- fixing a flexible piece (8) of a material that is not permeable to a decontamination gas, for example to hydrogen peroxide vapors, over the cover sheet, said flexible piece (8) being fixed to the cover sheet (4) by at least one of its edges (15) and comprising a free part (16), said free part (16) being positioned facing the window (6),
- placing the objects (2) that are to be packaged in a box (3), and
- fixing said cover sheet (4) over the box (3) in such a way as to seal this box imperviously.

15. The method as claimed in claim 14, **characterized in that** the flexible piece (8) is fixed to the exterior face of the cover sheet (4).

16. The method as claimed in claim 14, **characterized in that** the flexible piece (8) is fixed to the interior face of the cover sheet (4).

17. The use of the packaging (1) as claimed in one of claims 1 to 13 in a method for decontaminating this packaging using a decontamination gas, for example using hydrogen peroxide vapors.

18. The use of the packaging (1) as claimed in one of claims 1 to 13 for transporting syringe components, particularly syringe bodies (2) which are intended to be filled later with an active product or a medicinal product.

19. A sterilization and decontamination method using a packaging (1) as claimed in any one of claims 1 to 13, **characterized in that** it comprises the steps consisting in:
- placing the packaging (1) in a diffusion position during the sterilization process; and
- placing the packaging (1) in a non-diffusion position during the decontamination process.

## Patentansprüche

1. Verpackung (1) für den Transport von sterilen oder zu sterilisierenden Objekten, wobei die Verpackung (1) ein Behältnis (3) zur Aufnahme der sterilen oder zu sterilisierenden Objekte aufweist, sowie eine Abdeckfolie (4), die auf dem Behältnis abdichtend angebracht ist,
**dadurch gekennzeichnet, dass** die Abdeckfolie (4) aus einem Material besteht, das für ein Dekontaminations-Gas, bspw. Wasserstoffperoxiddämpfe, undurchlässig ist, und zumindest ein Fenster (6) aufweist, das durch ein Teil (7) aus einem selektiv dichten Material geschlossen ist, und dass es darüber hinaus zumindest ein flexibles Teil (8) aus einem für ein Dekontaminations-Gas, bspw. für Wasserstoffperoxiddämpfe, undurchlässigem Material aufweist, wobei das flexible Teil (8) an die Abdeckfolie (4) über zumindest einer seiner Ränder (15) angebracht ist, und einen freien Abschnitt (16) aufweist, wobei der freie Abschnitt (16) des Teils (8) frei beweglich ist zwischen einer Diffusionsposition, welche die Diffusion eines sterilisierenden Gases ins Innere des Behältnis (3) durch das Fenster (6) ermöglicht, und einer Position, in der die Diffusion von zumindest einem Teil des Dekontaminations-Gases, bspw. Wasserstoffperoxiddämpfe, ins Innere des Behältnis (3) durch das Fenster (6) verhindert wird.

2. Verpackung nach Anspruch 1, **dadurch gekennzeichnet, dass** das selektiv dichte Material ausgewählt ist aus Papier, Materialien, die auf natürlichen oder synthetischen Fasern basieren, Materialien, die auf Filamenten aus Polyethylen mit hoher Dichte oder anderen Polymeren basieren, welche insbesondere durch Hitze oder Druck verbunden sind, und deren Kombinationen.

3. Verpackung nach Anspruch 2, **dadurch gekennzeichnet, dass** das selektiv dichte Material ein Material ist, das auf Polyethylenfilamenten mit hoher Dichte basiert, die durch Hitze oder Druck verbunden sind.

4. Verpackung nach einem der vorstehenden Ansprüche, **dadurch gekennzeichnet, dass** das Behältnis (3) einen Rand (12) aufweist, der sich über die Abdeckfolie (4) erstreckt, und dass das flexible Teil (8) auf der äußeren Fläche (14) der Abdeckfolie angebracht ist.

5. Verpackung nach einem der Ansprüche 1 bis 3, **dadurch gekennzeichnet, dass** das flexible Teil (8) auf der inneren Fläche (9) der Abdeckfolie angebracht ist.

6. Verpackung nach einem der vorstehenden Ansprüche, **dadurch gekennzeichnet, dass** das flexible Teil (8) auf der Abdeckfolie (4) gegenüber dem Fenster (6) angebracht ist.

7. Verpackung nach einem der vorhergehenden Ansprüche, **dadurch gekennzeichnet, dass** das flexible Teil (8) eine Form aufweist, die ähnlich zu derjenigen des Fensters (6) ist, und dass die Oberfläche des flexiblen Teiles (8) größer ist als diejenige des Fensters (6), so dass in der Position, in der keine Diffusion möglich ist, das flexible Teil (8) das Fenster (6) über dessen gesamten Umfang überlappend abdeckt.

8. Verpackung nach einem der vorstehenden Ansprüche, **dadurch gekennzeichnet, dass** die Abdeckfolie (4) mehrere Fenster (6) aufweist, von denen jedes durch ein Teil (7) aus einem selektiv dichten Material verschlossen ist.

9. Verpackung nach Anspruch 8, **dadurch gekennzeichnet, dass** das flexible Teil (8) auf der Abdeckungsfolie (4) gegenüber den mehreren Fenster angebracht ist, und zwar derart, dass in der Position, in der keine Diffusion möglich ist, das flexible Teil (8) alle Fenster (6) durch Überlappung abdeckt.

10. Verpackung nach Anspruch 8, **dadurch gekennzeichnet, dass** mehrere flexible Teile (8) auf der Abdeckungsfolie (4) angebracht sind, wobei jeweils eines gegenüber einem entsprechenden Fenster (6) vorgesehen ist, und zwar derart, dass in der Position, in der keine Diffusion möglich ist, jedes flexible Teil (8) das Fenster (6), gegenüber welchem es angebracht ist, durch Überlappung abdeckt.

11. Verpackung nach einem der vorstehenden Ansprüche, **dadurch gekennzeichnet, dass** sie im Inneren des Behältnisses (3) zumindest eine Schicht (17) aufweist, die den Durchlass eines Dekontaminations-Gases, bspw. Wasserstoffperoxiddämpfe, beschränkt, oder zum Absorbieren eines Dekontaminations-Gases, bspw. Wasserstoffperoxiddämpfe.

12. Verpackung nach Anspruch 11, **dadurch gekennzeichnet, dass** die Schicht eine Form und Ausmaße aufweist, über welche sie entlang der Abdeckfolie platziert werden kann, und dass sich die Schicht in dieser Position zwischen der Abdeckfolie (4) und den in der Verpackung enthaltenen Objekten (2) erstreckt.

13. Verpackung nach einem der Ansprüche 11 oder 12, **dadurch gekennzeichnet, dass** die Schicht oder zumindest eine der Schichten über den ins Innere des Behältnisses eingebrachten Objekten vorgesehen ist, und zwar bevor die Abdeckungsfolie abdichtend angebracht wird, oder auf zu diesem Zweck vorgesehene Stützen, oder auf ein in dem Behältnis vorgesehenen Positionierteil für die Objekte.

14. Verfahren zur Herstellung einer Verpackung (1) gemäß einem der Ansprüche 1 bis 13, **dadurch gekennzeichnet, dass** es Schritte aufweist, die bestehen aus:
- Herstellen einer Abdeckfolie (4) aus einem für ein Dekontaminations-Gas, bspw. Wasserstoffperoxiddämpfe, undurchlässigem Material, wobei zumindest ein Fenster (6) in der Abdeckfolie angeordnet ist;
- Platzieren eines Teiles (7) aus einem selektiv dichten Material in das Fenster, so dass das Fenster durch das Material verschlossen wird,
- Anbringen eines flexibles Teiles (8) aus einem für ein Dekontaminations-Gas, bspw. Wasserstoffperoxiddämpfe, undurchlässigem Material auf der Abdeckfolie, wobei das flexible Teil (8) über zumindest einer seiner Ränder (15) an der Abdeckfolie (4) angebracht wird, und wobei es zumindest einen freien Abschnitt (16), aufweist, wobei der freie Abschnitt (16)gegenüber dem Fenster (6) positioniert ist,
- Platzieren der zu verpackenden Objekte (2) in ein Behältnis (3), und
- Anbringen der Abdeckfolie (4) auf dem Behältnis (3), derart, dass das Behältnis abdichtend verschlossen wird.

15. Verfahren nach Anspruch 14, **dadurch gekennzeichnet, dass** das flexible Teil (8) auf der äußeren Fläche der Abdeckungsfolie (4) angebracht wird.

16. Verfahren nach Anspruch 14, **dadurch gekennzeichnet, dass** das flexible Teil (8) auf der inneren Fläche der Abdeckfolie (4) angebracht wird.

17. Verwendung einer Verpackung (1) gemäß einem der Ansprüche 1 bis 13 in einem Verfahren zur Dekontamination der Verpackung durch ein Dekontaminations-Gas, bspw. durch Wasserstoffperoxiddämpfe.

18. Verwendung der Verpackung (1) gemäß einem der Ansprüche 1 bis 13, zum Transport von Bauelementen von Spritzen, insbesondere von Spritzenkörpern (2), die anschließend mit einem aktiven Produkt oder einem Medikament gefüllt werden sollen.

19. Verfahren zur Sterilisierung und zur Dekontamination, bei dem eine Verpackung (1) gemäß der Ansprüche 1 bis 13 eingesetzt wird, **dadurch gekennzeichnet, dass** es Schritte aufweist, die bestehen aus:
- Platzieren einer Verpackung (1) in eine Diffusionsposition während des Sterilisierungsprozesses; und
- Platzieren der Verpackung (1) in eine Position, in der keine Diffusion erfolgt, während des Dekontaminierungsprozesses.
